# EUROPÄISCHE PATENTANMELDUNG

(11) **EP 1 329 757 A1**
(43) Veröffentlichungstag der Anmeldung: **23.07.2003**
(21) Anmeldenummer: 03001138.1
(22) Anmeldetag: 21.01.2003
(51) Int. Cl.: G02B 21/00

(54) **Übertragungseinrichtung für ein Operationsmikroskop**

(30) Priorität: 22.01.2002 DE 10202125
(71) Anmelder: Leica Microsystems (Schweiz) AG, 9435 Heerbrugg (CH)
(72) Erfinder: Sander, Ulrich, Dr., 9445 Rebstein (CH)
(74) Vertreter: Reichert, Werner F., Dr.

(57) **Zusammenfassung**

Die Erfindung betrifft eine Übertragungseinrichtung zur Ansteuerung einer optischen Betrachtungseinheit, beispielsweise eines Operationsmikroskops, bei dem die Ansteuerung oder Funktionsüberprüfung für das Mikroskop (1), wie beispielsweise Zoom Fokus, Arbeitsabstand, Positionierung, Kippen, Schwenken, Video etc., per bidirektionaler - vorzugsweise elektromagnetischer - Strahlung (8) zwischen einer Steuerelektronik (3) mittels einer am Stativ (2) befestigten Sende-/Empfangseinheit (5) für das Mikroskop (1) erfolgt.

## Beschreibung

Die Erfindung betrifft eine Übertragungseinrichtung für eine optische Betrachtungseinheit, beispielsweise ein (Operations-)Mikroskop.

Bei den heute üblichen Operationsmikroskopen erfolgt die Übertragung der Daten für das Mikroskop - wie Zoom, Fokus, Arbeitsabstand, Positionierung, Kippen, Schwenken, Video etc. - per Elektrokabel, sodass eine Vielzahl von Kabeln im Mikroskop verlegt werden müssen.

Als Beispiele für die Reduktion von Verbindungskabeln können die räumliche Integration der Leistungs- und Datenleitungen, welche in EP 1 124 150 A1 offengelegt wurde, oder der "Foot-switch with radio control", Datenblätter der Fa. Steute Medizintechnik, Löhne (DE) (5 Seiten; vom 20.07.2000), angeführt werden. Auch mit Hilfe der CAN-Technologie wird die Anzahl der Kabel reduziert.

Die Fa. Maquet, Rastatt (DE) (Prospekt Reg. 6535.005.25.500 6.92 E&B), bietet mit dem "OP-System 1120" Infrarotsteuerungen zur Positionsveränderung von Operationstischen an.

Der Erfinder erkannte, dass diese bekannten Systeme nachteilig sind in Bezug auf die folgenden Punkte:
a) Durch die Kabel ergeben sich Einschränkungen in der Bewegungsfreiheit des Operationsmikroskops.
b) Aufwändige konstruktive Lösungen werden benötigt, um die Kabel bruchsicher und integriert im Stativ zu verstauen.
c) Krümmungsradien von Kabeln können nicht beliebig verkleinert werden. Damit werden Kräfte auf die Drehgelenke ausgeübt.
d) Aufwändige konstruktive Lösungen werden benötigt, um die Kabel bruchsicher und integriert im Stativ zu verstauen.

Aufgabe der Erfindung ist es nun, eine Übertragungseinrichtung zu schaffen, welche die Daten für das Mikroskop ohne die heute üblichen Strom- und Datenkabel von der Elektronik zum Mikroskop und den Steuereinrichtungen überträgt.

Gelöst wird diese Aufgabe durch die nachfolgend beschriebene Übertragungseinrichtung:

Die Steuer- und/oder Messdaten für das Mikroskop, wie beispielsweise Zoom, Fokus, Arbeitsabstand, Positionierung, Kippen, Schwenken, Video etc., werden im Betriebszustand von der Steuerelektronik mittels einer Sende-/Empfangseinheit per bidirektionaler - vorzugsweise elektromagnetischer - Strahlung zu einer am Mikroskop befestigten Sende/Empfangseinheit übermittelt. Dadurch kann ein kabelloses Operationsmikroskop mit Funkübertragung geschaffen werden.

Die Steuerelektronik und deren Sende-/Empfangseinheit kann nicht nur am Stativ befestigbar, sondern auch freistehend aufstellbar ausgebildet werden.

Die bidirektionale Funkübertragung kann sowohl als digitale oder analoge elektromagnetische Strahlung wie auch als Infrarot- oder Ultraschallstrahlung erfolgen.

Die bidirektionale Ausbildung der erfindungsgemäßen Funkübertragung ermöglicht in einer weiteren Ausgestaltungsform auch die Übermittlung der von konventionellen Handgriffen oder beispielsweise von einer Handgriff-Mouse mit Trackball oder von einem Mouse-Pad erzeugten Daten.

Um für den Patienten, den Chirurgen und/oder weitere elektronische Geräte schädliche Strahlungen zu vermeiden, werden die Sendeeinheiten gegenüber bestimmten Bereichen abgeschirmt. Mittels einer Richtstrahlübertragung kann außerdem der Strahlungskegel sehr schlank gehalten werden. Die Reichweite der Strahlung kann bewusst klein gehalten werden, sodass entfernte Fremdgeräte möglichst nicht beeinflusst werden können.

Durch die vorgängig beschriebene Übertragungseinrichtung werden die folgenden Verbesserungen erreicht:
- Keine Einschränkungen der Bewegungsfreiheit des Chirurgen und des Trägersystems für optische Instrumente.
- Mechanisch einfache konstruktive Lösung.
- Verwendbarkeit von Störungen an den Kabeln, die zum Ausfall des Gerätes führen können.
- Bei elektromagnetischer oder Ultraschallstrahlung keine Abschattungsgefahr wie bei Infrarot.

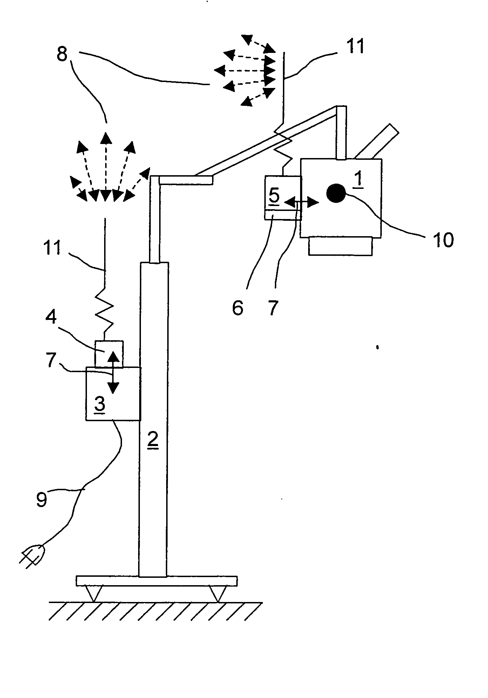

In der Zeichnung wird eine erfindungsgemäße und bevorzugte Übertragungseinrichtung schematisch dargestellt. Die von einer Steuerelektronik 3 des Mikroskops 1 erzeugten Daten 7, wie beispielsweise Zoom, Fokus, Arbeitsabstand, Positionierung, Kippen, Schwenken, Video-Übertragung etc., werden über eine mit der Steuerelektronik 3 verbundene Sende/Empfangseinheit 4 per elektromagnetischer Strahlung 8 auf eine am Mikroskop 1 befestigte Sende-/Empfangseinheit 5 übermittelt. Die Sende-/Empfangseinheit 4 der Steuerelektronik 3 ist beispielsweise an einem Stativ 2 befestigt und wird über eine Netzverbindung 9 der Steuerelektronik 3 gespeist. Die bidirektionale Funkübertragung 8 kann sowohl als digitale und/oder analoge elektromagnetische Strahlung, welche über Antennen 11 abgestrahlt - beispielsweise empfangen - wird und/oder als Ultraschallwelle erfolgen. Von der Sende-/Empfangseinheit 5 des Mikroskops 1 gehen Steuerdaten 7 zu Stellelementen des Mikroskops 1.

Um die Versorgungskabel der Sende-/Empfangseinheit 5 am Mikroskop 1 zu vermeiden, kann die Speisung dieser Sende-/Empfangseinheit 5 über einen Akku 6, der gegebenenfalls über Solarzellen aufladbar ist, beziehungsweise über Batterien erfolgen.

### Bezugszeichenliste

- 1: Mikroskop
- 2: Stativ
- 3: Steuerelektronik
- 4: Sende-/Empfangseinheit von (3)
- 5: Sende-/Empfangseinheit von (3) an (1)
- 6: Akku/Batterie(n)
- 7: Daten (Steuerdaten)
- 8: bidirektionale/elektromagnetische Strahlung (Funkübertragung)
- 9: Netzverbindung
- 10: Handgriff(e)
- 11: Antenne(n)

## Patentansprüche

1. Übertragungseinrichtung zur Ansteuerung oder Funktionsüberprüfung einer optischen Betrachtungseinheit, beispielsweise eines Operations-Mikroskops, **dadurch gekennzeichnet, dass** im Betriebszustand die Ansteuerung oder Funktionsüberprüfung für das Mikroskop (1) - wie beispielsweise Zoom, Fokus, Arbeitsabstand, Positionierung, Kippen, Schwenken, Video etc. - per bidirektionaler Strahlung (8) zwischen einer Steuerelektronik (3) mittels einer beispielsweise am Stativ (2) befestigten Sende-/Empfangseinheit (4) zu einer am Mikroskop (1) angeordneten Sende-/Empfangseinheit (5) für das Mikroskop (1) erfolgt.

2. Übertragungseinrichtung nach Anspruch 1, **dadurch gekennzeichnet, dass** die Sende-/Empfangseinheiten (4, 5) zur Übermittlung der Daten (7) zur Steuerung des Mikroskops (1) im Betriebszustand digitale und/oder analoge elektromagnetische Strahlung senden beziehungsweise empfangen.

3. Übertragungseinrichtung nach einem der vorhergehenden Ansprüche, **dadurch gekennzeichnet, dass** Sende-/Empfangseinheiten (4, 5) zur Übermittlung der Daten (7) zur Steuerung des Mikroskops (1) im Betriebszustand Lichtwellen - beispielsweise im IR-Bereich - senden beziehungsweise empfangen.

4. Übertragungseinrichtung nach Anspruch 1, **dadurch gekennzeichnet, dass** die Sende-/Empfangseinheiten (4, 5) zur Übermittlung der Daten (7) zur Steuerung des Mikroskops (1) im Betriebszustand Schallwellen - beispielsweise Ultraschallwellen - senden beziehungsweise empfangen.

5. Übertragungseinrichtung nach einem der vorhergehenden Ansprüche, **dadurch gekennzeichnet, dass** die Sende-/Empfangseinheiten (4, 5) zur Vermeidung unerwünschter Emissionen partiell abgeschirmt sind.

6. Übertragungseinrichtung nach einem der vorhergehenden Ansprüche, **dadurch gekennzeichnet, dass** die Übertragung der Steuerdaten (7) per Richtstrahl erfolgt.

7. Übertragungseinrichtung nach einem der vorhergehenden Ansprüche, **dadurch gekennzeichnet, dass** die Sende-/Empfangseinheit (4) wahlweise am Stativ (1) oder davon separiert installierbar ist.

8. Übertragungseinrichtung nach einem der vorhergehenden Ansprüche, **dadurch gekennzeichnet, dass** Schalter und Steuerelemente an Handgriffen (10) vorgesehen sind, die im Betriebszustand elektronische Signale - gegebenenfalls kabellos - an die Sende-/Empfangseinheiten (4, 5) weitergeben.

9. Übertragungseinrichtung nach einem der vorhergehenden Ansprüche, **dadurch gekennzeichnet, dass** die an Handgriffen vorhandenen Schalt- und Steuerelemente in Signalverbindung mit externen Geräten bringbar sind.

10. Übertragungseinrichtung nach einem der vorhergehenden Ansprüche, **dadurch gekennzeichnet, dass** die Energieversorgung der Sende-/Empfangseinheit (5) am Mikroskop (1) durch - vorzugsweise wiederaufladbare - Batterien (6) erfolgt, gegebenenfalls in Verbindung mit Solarzellen.
